# EUROPÄISCHE PATENTSCHRIFT

(11) **EP 1 988 783 B1**
(45) Veröffentlichungstag und Bekanntmachung des Hinweises auf die Patenterteilung: **12.08.2015**
(21) Anmeldenummer: 07726450.5
(22) Anmeldetag: 20.02.2007
(51) Int. Cl.: A22C 25/00, A22C 7/00, A23L 1/00, A23L 1/216, A23L 1/212, A23L 3/36, A23P 1/10

(54) **VERFAHREN ZUM HERSTELLEN VON GEFORMTEN LEBENSMITTELN AUS GEMÜSE-, FLEISCH- UND ANDEREN LEBENSMITTELEINZELSTÜCKEN**
METHOD FOR PRODUCING MOLDED FOOD ITEMS FROM INDIVIDUAL PIECES OF VEGETABLES, MEAT OR OTHER FOOD
PROCÉDÉ DE FABRICATION D'ALIMENTS MOULÉS À PARTIR DE LÉGUMES, DE VIANDE ET D'AUTRES ALIMENTS INDIVIDUELS

(30) Priorität: 20.02.2006 DE 102006008132; 04.05.2006 DE 102006021139
(43) Veröffentlichungstag der Anmeldung: 12.11.2008
(73) Patentinhaber: Nienstedt GmbH, 45721 Haltern am See (DE)
(72) Erfinder: GRONEBERG-NIENSTEDT, Petra, 45721 Haltern am See (DE); GUTMANN, Michael, 45721 Haltern am See (DE)
(74) Vertreter: Bungartz, Klaus Peter
(86) Internationale Anmeldenummer: PCT/EP2007/051629
(87) Internationale Veröffentlichungsnummer: WO 2007/096365

(56) Entgegenhaltungen:
- WO-A-2006/053601
- FR-A1- 2 847 427
- US-A- 4 626 436
- US-A- 4 973 492
- US-A- 5 223 297
- DATABASE FSTA [Online] INTERNATIONAL FOOD INFORMATION SERVICE (IFIS), FRANkFURT-MAIN, DE; ANONYMOUS: "Finger foods - new process offers exciting possibilities." XP002435342 Database accession no. 86-3-10-g0028 & ANONYMOUS: "Finger foods - new process offers exciting possibilities." FOOD REVIEW, Bd. 13, Nr. 1, 1986, Seite 19,

## Beschreibung

Die Erfindung betrifft ein Verfahren zum Herstellen von zubereitungsfertigen Lebensmitteln mit einer gewünschten Form aus Gemüse oder Obst umfassenden Einzelstücken in Kombination mit weiteren, von Gemüse, Obst, Fleisch, Fisch, sonstigen Meeresfrüchten, Geflügel, Wild, Gebäck gebildeten Teilen oder aller dieser Inhaltsstoffe, mit den Schritten:
- Aussortieren zu großer Produkte,
- Zusammenstellen der Inhaltsstoffe zu Eingangsprodukten aus tiefgefrorenen Einzelstücken,
- kontinuierliches oder getaktetes Einbringen der Eingangsprodukte in wenigstens ein Formnest und
- Herstellen der gewünschten Form des Lebensmittels durch Pressen der gefrorenen Eingangsprodukte in dem Formnest zu einem gefrorenen Endprodukt, das back- oder kochfertig ist.

Aus der US 5 223 297 A ist ein Verfahren zum Herstellen von geformten Lebensmitteln, zum Beispiel Zwiebelringen bekannt. Hierbei werden gefrorene Zwiebelstücke über eine Förderschnecke einer Form zugeführt und anschließend zu den Zwiebelringen verpresst. Mit diesem Verfahren lassen sich gepresste Lebensmittel herstellen die dann tiefgefroren oder aufgetaut weiterverarbeitet werden können.

Aus der US 4 626 436 A ist ein Verfahren bekannt, bei denen aus unterschiedlichen Lebensmitteln, zum Beispiel auch Gemüse oder Früchten, tiefgefrorene Blöcke einer vorbestimmten Größe hergestellt werden, die anschließend zur Herstellung des Endproduktes durch einen Sägeschritt zerlegt werden. Dieses Verfahren eignet sich besonders zur Herstellung von Stücken einer einheitlichen Größe in Verbindung mit Gemüse, Fisch- oder Geflügelprodukten.

Aus der US 4 973 492 A ist ein Verfahren zum Herstellen von Grillbratlingen aus einer Mischung aus Fleisch und einer Grillsauce nahe der Gefriertemperatur von Wasser bekannt, wobei das Fleisch mit der Grillsauce vermischt wird, anschließend in die Form eines flachen Bratlings gepresst und nachfolgend auf die Lagertemperatur eingefroren wird.

Die WO 2006/053601 wiederum zeigt ein Verfahren zum Herstellen von geformten Fleischportionen die in der Form dem Gewicht weit gehend gleich sind. Hierzu wird Fleisch in einer Form gepresst nachdem es zuvor auf das gewünschte Zielgewicht beschnitten wurde.

Aus der Fisch-, Fleisch oder Geflügelverarbeitung ist es weiterhin bekannt, gefrorene Stücke des Lebensmittels zu einem geformten Endprodukt zu verarbeiten. Ein solches Verfahren wird in der WO 97/10717 A1 beschrieben. Hier werden Fleischstücke schon in der später gewünschten Form aus einem plattenartigen Material unter Druck geformt.

Aus der EP 0 168 909 A2 ist bekannt, einen gefrorenen Block aus Gemüse oder Früchten über das Shape-Verfahren herzustellen. Bei diesem Verfahren werden gefrorene Produkte verarbeitet, um hieraus zunächst Blöcke und später einzelne Portionen eines Lebensmittels herzustellen. Wesentliche Anwendung dieses Verfahrens ist beispielsweise die Herstellung von portionierten Spinatstücken, die dann aus der Verpackung portionsweise entnommen werden können. Nach dem Auftauen dieser Portionsstücke soll das Produkt dann auf übliche Weise zubereitet werden, wobei der während des Gefrierens erfolgte Verbund infolge der Zubereitung wieder aufgelöst wird und das Produkt in ein verzehrfertiges Lebensmittel zerfällt.

Der Nachteil der oben genannten Verfahren besteht darin, dass die Anwendungen auf die Herstellung einzelner bestimmter Lebensmittel gerichtet sind. Zubereitungsfertige Lebensmittelmischungen mit einer möglichst großen Variabilität der Inhaltsstoffe lassen sich jeweils nicht herstellen.

Neben den genannten Verfahren sind weitere Verfahren bekannt, mit denen Kartoffelprodukte aus gestampften Kartoffeln hergestellt werden können. So offenbart die US 4 276 314 A ein Verfahren zum Herstellen von so genannten Hash-Brown-Potatoes, bei dem gestampfte Kartoffelprodukte vor dem Tieffrieren in die gewünschte Produktform gebracht und anschließend eingefroren werden. Nach dem Wiederauftauen werden diese Produkte dann frittiert und verzehrfertig zubereitet. Dieses Verfahren eignet sich nicht für die Herstellung von Produkten, die nur durch das eigentliche Shapen (=Formen unter Druck eine Pressstempels in einer Formmulde) geformt werden sollen, sondern das Verfahren erfordert einen zusätzlichen äußeren Verbund bzw. einen Frittiervorgang, der schnell eine äußere Hülle mit entsprechender Festigkeit herzustellen vermag, um so das Zerfallen des Produktes zu vermeiden.

Es ist somit Aufgabe der Erfindung, ein Verfahren zum Herstellen von zubereitungsfertigen oder sogar verzehrfertigen Lebensmitteln mit einer möglichst großen Variabilität bereitzustellen.

Diese Aufgabe wird nach der Erfindung dadurch gelöst, dass das Verfahren die Schritte Zusammenfassung einer ersten Mischung von Einzelstücken zu einem ersten Vorprodukt und Zusammenfassung wenigstens einer zweiten Mischung von Einzelstücken zu einem zweiten Vorprodukt und anschließendes Einlegen aller Vorprodukte in das Formnest und Verpressen zu dem Lebensmittel umfasst, wobei das erste Vorprodukt einen konvexen Abschnitt aufweist, in den das zweite Vorprodukt eingelegt wird.

Ein Merkmal der Erfindung ist die Tatsache, dass die gefrorenen Eingangsprodukte dadurch zu einem back- oder kochfertigen gefrorenen Endprodukt verarbeitet werden können, dass sie im gefrorenen Zustand in dem Formnest auf die gewünschte Form gepresst werden. Die so hergestellten Produkte können dann im Rahmen einer Zubereitung, etwa in einem Ofen oder auch in einer Pfanne, auch ohne weitere Behandlung unter Beibehaltung der Form gegart werden. Dabei ist es möglich, dass die Produkte so hergestellt werden, dass sie während der weiteren Zubereitung bewusst zerfallen sollen um den Eindruck einer Zubereitung aus Lebensmitteln zu erwecken, die in der naturgewachsenen oder einer ähnlichen Form verarbeitet wurden.

Das erfindungsgemäße Verfahren ist auf die Herstellung aller Arten von Lebensmitteln aus gefrorenen Einzelstücken anwendbar. Als Einzelstücke können Gemüse, Obst, Fleisch, Geflügel, Wild oder Fisch bzw. sonstige Meeresfrüchte verwendet werden. Die Einzelstücke können auch eine Kombination von Teilen oder aller dieser Inhaltsstoffe sein. In einem ersten Schritt werden die notwendigen Einzelstücke zusammengestellt, was insbesondere ein Sortieren nach der Größe und dem Gewicht mit sich bringt.

Die Eingangsprodukte dürfen natürlich nur so groß sein, dass sich aus ihnen die gewünschten Endprodukte herstellen lassen. Auch wenn grundsätzlich die Anwendung der Erfindung in Verbindung mit zerstampften oder gemahlenen Produkten möglich ist, wird doch ein wesentlicher Anwendungsbereich der Erfindung darin bestehen, dass Eingangsprodukte verwendet werden, die kleiner sind als die späteren Endprodukte, aber immer noch ganze Stücke des ursprünglichen Materials darstellen. Die so ausgewählten und gefrorenen Eingangsprodukte werden dann durch Verpressen in dem Formnest zu dem Endprodukt geformt, wobei während der gesamten Verarbeitung die Inhaltsstoffe gefroren bleiben.

Bei einigen Produkten hat es sich gezeigt, dass alleine durch das Verpressen der Eingangsprodukte unter tiefen Temperaturen die Produkte derart fest miteinander verbunden bleiben, dass ein Zerfallen während der späteren Zubereitung, die natürlich ein Auftauen der Endprodukte mit sich bringen wird, vermieden werden kann. Die Verbindung der einzelnen Einzelstücke kann jedoch vor dem Einfrieren der Einzelstücke oder auch vor dem Verpressen und sogar noch anschließend dadurch verbessert werden, dass zusätzliche Haftmittel zugefügt werden. Im einfachsten Fall kann dies eine Flüssigkeit, insbesondere Wasser oder eine proteinhaltige Flüssigkeit sein, die den Verbund der Einzelstücke untereinander verbessert.

Vor dem Zusammenstellen der Eingangsprodukte zu den gefrorenen Einzelstücken werden die zu großen Produkte, ebenso wie die zu kleinen Eingangsprodukte aussortiert. Zu große Produkte können entweder einer anderweitigen Verarbeitung zugeführt oder einem Zerkleinerungsprozess unterzogen werden, so dass sie dem eigentlichen Verfahren wieder zuführbar sind. Zu kleine Produkte werden üblicherweise anderweitig weiterverarbeitet.

Die Haftkraft der einzelnen Einzelstücke untereinander kann ferner dadurch erhöht werden, dass die Einzelstücke vor Eingabe in die Formnester an- oder aufgetaut werden. So können bei nachfolgendem Tiefgefrieren vor dem eigentlichen Verpressen bereits klebrige Verbindungen durch Festfrieren der einzelnen Teile aneinander hergestellt werden. Auch ist es möglich, die gefrorenen oder angetauten Eingangsprodukte mit zusätzlichem Wasser oder anderen Flüssigkeiten zu besprühen.

Die erfindungsgemäße Ausgestaltung des Verfahrens ermöglicht nun die Herstellung eines Verbundes von mehreren Vorprodukten zu einem verzehrfertigen Endprodukt. Hierzu wird ein Vorprodukt einer ersten Art aus einer Mischung von Einzelstücken hergestellt und kann dann zum Beispiel in einem Vorformprozess bereits vorgepresst, so dass dann in einem zweiten Schritt eine auf gleiche Weise hergestellte Mischung eines zweiten Vorproduktes mit diesem ersten Vorprodukt zusammenverpresst oder anderweitig zusammengefügt werden kann. So kann etwa ein Reisschälchen als erstes Vorprodukt hergestellt werden, während eine Soßen-Gemüsemischung oder auch eine Fleischeinlage als zweites Vorprodukt verwendet wird.

Das erste Vorprodukt weist eine konkave Form aufweisen, während das zweite Vorprodukt in die schalenartige Aufnahme des konkaven ersten Vorproduktes eingelegt wird. Je nach Wunsch des Anwenders kann das erste Vorprodukt auch so ausgestaltet sein, dass es während des Garprozesses zerfällt, so dass das formstabile zweite Vorprodukt dann in einem Bett des zerfallenen ersten Vorproduktes zum Liegen kommt. In einem Beispiel kann etwa das erste Vorprodukt der bereits genannte Reisrand sein, während das zweite Vorprodukt dann zum Beispiel eine Mischung aus Gemüse und Obst ist. Auch ein Fleischprodukt kann so innerhalb des Reisrandes angeordnet werden.

Die Einzelstücke können vor dem Verpressen in dem Formnest mit weiteren Zugaben versehen werden. Dies kann beispielsweise eine Marinade oder auch ein Pulvergewürz sein. Um die nach dem Verpressen hergestellten Endprodukte einfacher aus den Formnestern entnehmen zu können, ist es möglich, die Formnester leicht anzuwärmen, so dass die äußere Schicht des Endproduktes aufschmilzt und so ein Festfrieren an der Wandung des Formnestes vermieden wird.

Bevorzugt wird mit dem Verfahren überwiegend oder vollständig Gemüse oder Obst in Stücken verarbeitet werden. Die Stücke werden tief gefroren, der Größe nach sortiert und anschließend im tief gefrorenem Zustand entweder ohne oder mit Zugabe von weiteren Haftvermittlern oder von weiteren Geschmacksverbesserem zu einem zubereitungsfertigen, formstabilen Endprodukt verpresst.

## Patentansprüche

1. Verfahren zum Herstellen von zubereitungsfertigen Lebensmitteln mit einer gewünschten Form aus Gemüse oder Obst umfassenden Einzelstücken in Kombination mit weiteren, von Gemüse, Obst, Fleisch, Fisch, sonstigen Meeresfrüchten, Geflügel, Wild, Gebäck gebildeten Teilen oder aller dieser Inhaltsstoffe, mit den Schritten:
• Aussortieren zu großer Produkte,
• Zusammenstellen der Inhaltsstoffe zu Eingangsprodukten aus tiefgefrorenen Einzelstücken,
• kontinuierliches oder getaktetes Einbringen der Eingangsprodukte in wenigstens ein Formnest und
• Herstellen der gewünschten Form des Lebensmittels durch Pressen der gefrorenen Eingangsprodukte in dem Formnest zu einem gefrorenen Endprodukt, das back- oder kochfertig ist,
**dadurch gekennzeichnet, dass**
• eine erste Mischung von Einzelstücken zu einem ersten Vorprodukt zusammengefasst und wenigstens eine zweite Mischung von Einzelstücken zu einem zweiten Vorprodukt zusammengefasst werden und anschließend alle Vorprodukte in das Formnest eingelegt und zusammen zu dem Lebensmittel verpresst werden, wobei
• das erste Vorprodukt einen konkaven Abschnitt aufweist, in den das zweite Vorprodukt eingelegt wird.

2. Verfahren nach dem vorhergehenden Anspruch, **dadurch gekennzeichnet, dass** die Einzelstücke vor dem Einbringen in das Formnest sortiert werden und Einzelstücke, die zu klein sind, aussortiert werden.

3. Verfahren nach dem vorhergehenden Anspruch, **dadurch gekennzeichnet, dass** zu große Einzelstücke nach dem Aussortieren zerkleinert und anschließend dem Verfahren zusammen mit den nachfolgenden Einzelstücken wieder zugeführt werden.

4. Verfahren nach einem der vorhergehenden Ansprüche, **dadurch gekennzeichnet, dass** den Einzelstücken vor dem Einbringen in die Formnester eine Flüssigkeit, insbesondere Wasser oder eine proteinhaltige Flüssigkeit zugesetzt werden, um den Verbund der Einzelstücke zu verbessern.

5. Verfahren nach einem der vorhergehenden Ansprüche, **dadurch gekennzeichnet, dass** die Einzelstücke vor der Eingabe in die Formnester an- oder aufgetaut werden und anschließend, vor dem Verpressen wieder gefroren werden.

6. Verfahren nach Anspruch 1, **dadurch gekennzeichnet, dass** das erste und/oder das zweite Vorprodukt aus gefrorenen Einzelstücken aufgebaut wird, die durch Besprühen oder Antauen klebrig gemacht wurden und dass das erste und/oder das zweite Vorprodukt nach dem Formen wieder tief gefroren werden.

7. Verfahren nach einem der vorhergehenden Ansprüche, **dadurch gekennzeichnet, dass** die Einzelstücke vor dem Verpressen in dem Formnest, insbesondere durch Zugabe einer Marinade oder eine Pulvergewürzes gewürzt werden.

8. Verfahren nach einem der vorhergehenden Ansprüche, **dadurch gekennzeichnet, dass** die Wandungen der Formnester zum leichteren Entformen der gepressten Lebensmittel vor dem Entformen leicht angewärmt werden.

9. Verfahren zum Herstellen eines verzehrfertigen Lebensmittels, **dadurch gekennzeichnet, dass** zunächst ein zubereitungsfertiges Lebensmittel nach einem der vorhergehenden Ansprüche hergestellt wird und anschließend dieses zubereitungsfertiges Lebensmittel im gefrorenen Zustand einem Garprozess, insbesondere einem Ofen oder einer Fritteuse, zugeführt wird.

10. Verfahren nach einem der vorstehenden Ansprüche, **dadurch gekennzeichnet, dass** die Einzelstücke zumindest teilweise Stücke von Kartoffeln sind.

## Claims

1. A method for producing ready-to-use food having a desired shape of single pieces comprising vegetables or fruit in combination with further parts formed from vegetables, fruit, meat, fish, other seafood, poultry, game, pastries or all of these ingredients, comprising the steps:
• sorting out products, which are too large,
• assembling the ingredients to form input products of deep-frozen single pieces,
• continuous or clocked introduction of the input products into at least one mold cavity and
• producing the desired shape of the food by pressing the frozen input products in the mold cavity to form a frozen end product, which is ready-to-bake or ready-to-cook,
**characterized in that**
• a first mixture of single pieces is combined to form a first pre-product and at least a second mixture of single pieces is combined to form a second pre-product and all pre-products are subsequently placed into the mold cavity and are pressed to form the food, wherein
• the first pre-product encompasses a concave section, into which the second pre-product is placed.

2. The method according to the preceding claim, **characterized in that** the single pieces are sorted prior to being introduced into the mold cavity and single pieces, which are too small, are sorted out.

3. The method according to the preceding claim, **characterized in that** single pieces, which are too large, are comminuted after being sorted out and are subsequently supplied to the method again together with the following single pieces.

4. The method according to one of the preceding claims, **characterized in that** a liquid, in particular water or a protein-containing liquid is added to the single pieces prior to introduction into the mold cavities, so as to improve the bond of the single pieces.

5. The method according to one of the preceding claims, **characterized in that** the single pieces are thawed slightly or completely prior to inputting into the mold cavities and are subsequently frozen again prior to the pressing.

6. The method according to claim 1, **characterized in that** the first and/or the second pre-product is made of frozen single pieces, which were made sticky by spraying or slightly thawing, and that the first and/or the second pre-product is deep-frozen again after the forming.

7. The method according to one of the preceding claims, **characterized in that** the single pieces are seasoned prior to the pressing in the mold cavity, in particular by adding a marinade or a powder seasoning.

8. The method according to one of the preceding claims, **characterized in that** the walls of the mold cavities are heated slightly prior to the demolding so as to facilitate demolding of the pressed food.

9. A method for producing a ready-to-eat food, **characterized in that** a ready-to-use food according to one of the preceding claims is produced initially and that this ready-to-use food is subsequently supplied to a cooking process, in particular to an oven or a deep-fryer, in the frozen state.

10. The method according to one of the preceding claims, **characterized in that** the single pieces are at least partially pieces of potatoes.

## Revendications

1. Procédé destiné à confectionner des produits alimentaires prêts à cuisiner d'une forme souhaitée en parts individuelles comprenant des légumes ou des fruits en association avec d'autres pièces formées de légumes, de fruits, de viande, de poisson, de divers fruits de mer, de volaille, de gibier, de produits de boulangerie ou de l'ensemble desdits ingrédients, avec les étapes :
• tri des produits trop gros
• assemblage des ingrédients en produits de départ en parts individuelles surgelées,
• introduction continue ou cyclique des produits de départ dans au moins une cavité de moule et
• confection de la forme souhaitée du produit alimentaire par compactage des produits de départ gelés dans la cavité de moule en un produit final gelé, qui est prêt à cuire ou à cuire au four, **caractérisé en ce**
• **qu'**on assemble un premier mélange de parts individuelles en un premier produit semi-fini et au moins un deuxième mélange de parts individuelles en un deuxième produit semi-fini et on pose ensuite l'ensemble des produits semi-finis dans la cavité de moule et on les compresse ensemble pour obtenir le produit alimentaire,
• le premier produit semi-fini comportant une partie concave dans laquelle on pose le deuxième produit semi-fini.

2. Procédé selon l'une quelconque des revendications précédentes, **caractérisé en ce qu'**on trie les parts individuelles avant l'introduction dans la cavité de moule et on élimine les parts individuelles qui sont trop petites.

3. Procédé selon l'une quelconque des revendications précédentes, **caractérisé en ce qu'**après leur élimination, on fractionne les parts individuelles trop grosses et on les ramène ensuite dans le procédé, ensemble avec les parts individuelles suivantes.

4. Procédé selon l'une quelconque des revendications précédentes, **caractérisé en ce qu'**avant leur introduction dans les cavités de moule, on rajoute aux parts individuelles un liquide, notamment de l'eau ou un liquide protéiné, pour améliorer la liaison des parts individuelles.

5. Procédé selon l'une quelconque des revendications précédentes, **caractérisé en ce qu'**avant de les introduire dans les cavités de moule, on fait dégeler légèrement ou décongeler les parts individuelles et ensuite, on les recongèle avant le compactage.

6. Procédé selon la revendication 1, **caractérisé en ce qu'**on élabore le premier et/ou le deuxième produit semi-fini à partir de parts individuelles congelées, que l'on a rendu gluantes par vaporisation ou dégel léger et **en ce qu'**après le moulage, on recongèle le premier et/ou le deuxième produit semi-fini.

7. Procédé selon l'une quelconque des revendications précédentes, **caractérisé en ce qu'**avant le compactage dans la cavité de moule, on assaisonne les parts individuelles notamment par ajout d'une marinade ou d'une épice en poudre.

8. Procédé selon l'une quelconque des revendications précédentes, **caractérisé en ce que** pour un démoulage facilité des produits alimentaires compressés, on chauffe légèrement les parois des cavités de moule avant le démoulage.

9. Procédé destiné à confectionner un produit alimentaire prêt à consommer, **caractérisé en ce qu'**on confectionne d'abord un produit alimentaire prêt à cuire et on amène ensuite ledit produit alimentaire prêt à cuire à l'état congelé à un processus de cuisson, notamment dans un four ou dans une friteuse.

10. Procédé selon l'une quelconque des revendications précédentes, **caractérisé en ce que** les parts individuelles sont au moins en partie des morceaux de pommes de terre.
